# EUROPEAN PATENT APPLICATION

(11) **EP 4 449 993 A1**
(43) Date of publication of application: **23.10.2024**
(21) Application number: 23168893.8
(22) Date of filing: 20.04.2023
(51) Int. Cl.: A61B 6/03, A61B 6/00, G06T 7/00

(54) **DETERMINING A STATUS OF VASCULAR DISEASE**

(71) Applicant: Koninklijke Philips N.V., 5656 AG Eindhoven (NL)
(72) Inventor: VEMBAR, Manindranath, Eindhoven (NL); GRASS, Michael, Eindhoven (NL); HAASE, Hans Christian, 5656AG Eindhoven (NL); VAN DER HORST, Arjen, Eindhoven (NL); NICKISCH, Hannes, Eindhoven (NL); SCHMITT, Holger, Eindhoven (NL)
(74) Representative: Philips Intellectual Property & Standards

(57) **Abstract**

A computer-implemented method of determining a status of vascular disease, is provided. The method includes: determining, from CT data, a spatial distribution of X-ray attenuation values across one or more concentric layers of perivascular adipose tissue, PVAT, surrounding a vessel; and analyzing the spatial distribution of X-ray attenuation values to provide a spatial distribution of disease status values (140) representing a status of vascular disease around the portion of the vessel. A graphical representation (150a, 150b) of the spatial distribution of disease status values (140) is outputted. Alternatively, or additionally, a predicted time at which the status of vascular disease around the portion of the vessel is expected to reach a predefined status, is outputted. The predicted time is predicted based on the spatial distribution of disease status values (140).

## Description

### TECHNICAL FIELD

The present disclosure relates to determining a status of vascular disease. A computer-implemented method, a computer program product, and a system, are disclosed.

### BACKGROUND

Vascular disease refers to a range of conditions that affect the circulatory system, or in other words, the blood vessels that circulate blood around the body. Some types of vascular disease affect the arteries, whereas other types of vascular disease affect the veins. Vascular disease can affect various parts of the body, including the heart, and also peripheral regions such as the leg, and so forth. For instance, Coronary Artery Disease "CAD" refers to pathologies of the coronary arteries and to the deterioration of their ability to transport oxygenated blood to the heart muscle. In the case of CAD, ultimately, the lack of oxygen supply results in myocardial ischemia, whose symptoms can include shortness of breath, angina, or even myocardial infarction.

Various imaging-based biomarkers have been developed for use in predicting risks relating to vascular disease. One such biomarker relates to the detection of inflammation in perivascular tissue "PVAT", and is disclosed the document WO 2016/024128 A1. This document defines methods for volumetric characterization of perivascular adipose tissue using data collected by computed tomography "CT" scanning. The volumetric characterization of perivascular adipose tissue allows the inflammatory status of underlying blood vessels to be established by CT scanning. This is of use in the diagnosis, prognosis and treatment of coronary and vascular disease.

Another document by Antoniades C., et al., "State-of-the-art review article. Atherosclerosis affecting fat: What can we learn by imaging perivascular adipose tissue?", J. Cardiovasc. Comput. Tomogr. 2019 Sep-Oct; 13(5):288-296, describes a biomarker that is derived from CT attenuation in PVAT surrounding the human coronary arteries. This document discloses a biomarker designed to capture spatial changes in PVAT's attenuation around the human coronary arteries, the Fat Attenuation Index "FAI". The FAI has predictive value in stable patients for cardiac mortality and non-fatal heart attacks.

PVAT surrounds the coronary arteries and is found within, or contiguous with, the Tunica adventitia layer, i.e. in the layer outside the Tunica media layer. The extent of PVAT in a direction extending radially outwards with respect to the vessel centerline is approximately equal to the diameter of the artery. PVAT is identifiable in CT images via its elevated level of X-ray attenuation just outside the vessel wall.

As described in the document by Antoniades C., et al., cited above, in the absence of vessel inflammation, the X-ray attenuation of PVAT decreases with increasing radial distance away from the vessel wall. By contrast, in the presence of vessel inflammation, relatively higher X-ray attenuation values are found in PVAT. This gives rise to a relatively higher X-ray attenuation values in the vicinity of the vessel wall, followed by a relatively steeper gradient with increasing radial distance away from the vessel wall. This document describes the evaluation of a biomarker for a segment of a vessel, the Fat Attenuation Index "FAI". The FAI quantifies a weighted measure of attenuation in concentric 1 millimeter-layers of perivascular tissue around the human arterial wall, capturing the respective perivascular attenuation gradients, reflecting the changes in PVAT biology that occur as a result of vascular inflammation. The FAI has predictive value in stable patients for cardiac mortality and non-fatal heart attacks.

In the document WO 2016/024128 A1, cited above, another biomarker for CAD, referred-to as Volumetric Perivascular Characterization Index "VPCI-i", is disclosed for the prediction of the presence of CAD. The biomarker VPCI-i, is calculated for a section of a vessel from a plot of the radiodensity change of perivascular adipose tissue around the right coronary artery with respect to distance from the outer wall of the vessel. An area under the curve technique is used to determine a value of the VPCI-i, for the vessel section, and to thereby ascertain the presence of CAD.

However, there remains a need to improve the evaluation of the status of vascular disease for a vessel from measurements X-ray attenuation in PVAT.

### SUMMARY

According to one aspect of the present disclosure, a computer-implemented method of determining a status of vascular disease, is provided. The method includes:
receiving computed tomography, CT, data representing a portion of a vessel;
determining, from the CT data, a spatial distribution of X-ray attenuation values across one or more concentric layers of perivascular adipose tissue, PVAT, surrounding the portion of the vessel; analyzing the spatial distribution of X-ray attenuation values across the one or more concentric layers to provide a spatial distribution of disease status values representing a status of vascular disease around the portion of the vessel; and
outputting a graphical representation of the spatial distribution of disease status values and/or outputting a predicted time at which the status of vascular disease around the portion of the vessel is expected to reach a predefined status, the predicted time being predicted based on the spatial distribution of disease status values.

In the above method, the provision of a spatial distribution of disease status values representing a status of vascular disease around the portion of the vessel, provides detailed information on the status of disease. This is in contrast to the provision of a single value of a biomarker for a portion of a vessel. The additional information that is provided by the spatial distribution of disease status values, may for instance be used to track changes in the disease at specific locations, thereby facilitating improved decision-making on the treatment or monitoring of the vessel. Similarly, since the outputted predicted time at which the status of vascular disease around the portion of the vessel is expected to reach a predefined status, is predicted based on the spatial distribution of disease status values, a more reliable predicted time can be provided.

Further aspects, features, and advantages of the present disclosure will become apparent from the following description of examples, which is made with reference to the accompanying drawings.

### BRIEF DESCRIPTION OF THE DRAWINGS

Fig. 1 is a schematic diagram illustrating an example of a portion of a vessel 120 including perivascular adipose tissue, PVAT, surrounding the vessel, in accordance with some aspects of the present disclosure.
Fig. 2 is a flowchart illustrating an example of a computer-implemented method of determining a status of vascular disease, in accordance with some aspects of the present disclosure.
Fig. 3 is a schematic diagram illustrating an example of a system 200 for determining a status of vascular disease, in accordance with some aspects of the present disclosure.
Fig. 4 is a schematic diagram illustrating an example of a portion of a vessel 120 including one or more concentric layers 130_{1..n} of perivascular adipose tissue, PVAT, surrounding the vessel, in accordance with some aspects of the present disclosure.
Fig. 5 is a schematic diagram illustrating an example of a technique for determining S 120 a spatial distribution of X-ray attenuation values across one or more concentric layers 130_{1..n} of PVAT, in accordance with some aspects of the present disclosure.
Fig. 6 illustrates a) an example of a graph of X-ray attenuation values along a path extending in a radial direction, R, from a vessel centerline, CL, and b) an example of a normalized graph of the X-ray attenuation values illustrated in a), in accordance with some aspects of the present disclosure.
Fig. 7 is a schematic diagram illustrating a first example of a graphical representation 150a of a spatial distribution of disease status values 140, in accordance with some aspects of the present disclosure.
Fig. 8. is a schematic diagram illustrating a second example of a graphical representation 150b of a spatial distribution of disease status values 140, in accordance with some aspects of the present disclosure.
Fig. 9 is a schematic diagram illustrating an example of a graphical representation of mutually registered spatial distributions of disease status values 140 representing the status of vascular disease around the portion of the vessel 120 at two different points in time t₀, t₁.
Fig. 10 is a schematic diagram illustrating an example of a graphical representation of a spatial distribution of disease status values representing a change in the status of vascular disease around the portion of the vessel 120 between two points in time t₀, t₁, in accordance with some aspects of the present disclosure.

### DETAILED DESCRIPTION

Examples of the present disclosure are provided with reference to the following description and Figs. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a computer implemented method, may be implemented in a computer program product, and in a system, in a corresponding manner.

In the following description, reference is made to a computer-implemented method of determining a status of vascular disease. In some examples, reference is made to vascular disease in the form of coronary artery disease, "CAD". However, in general it is to be appreciated that the methods are not limited to this type of vascular disease. The methods may also be used to determine a status of vascular disease in other parts of the body than the heart. The methods may also be used to determine a status of vascular disease in other types of blood vessels than an artery. In general, the methods may be used to determine a status of vascular disease in any anatomical region, and the vessel may be any type of vessel. Thus, the vessel may be an artery, or a vein, and the artery, or vein, may be disposed in any location in the body, such as in the heart, the brain, or in a peripheral region such as in the arm, the leg, and so forth.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid state memories, magnetic tape, removable computer disks, random access memory "RAM", read-only memory "ROM", rigid magnetic disks and optical disks. Current examples of optical disks include compact diskread only memory "CD-ROM", compact disk-read/write "CD-R/W", Blu-Ray^{™} and DVD.
As mentioned above, there remains a need to improve the evaluation of the status of vascular disease for a vessel from measurements X-ray attenuation in PVAT.

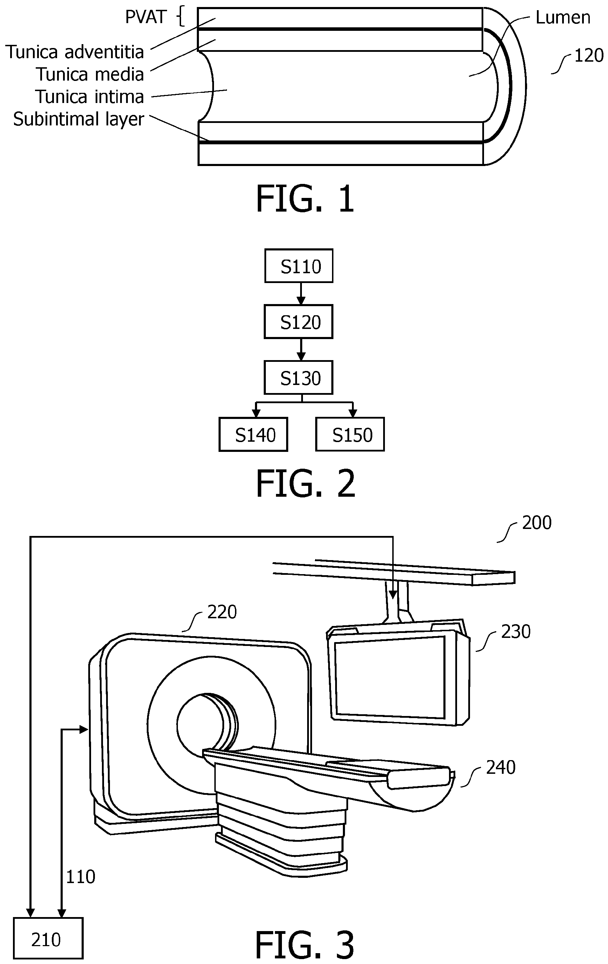

Fig. 1 is a schematic diagram illustrating an example of a portion of a vessel 120 including perivascular adipose tissue, PVAT, surrounding the vessel, in accordance with some aspects of the present disclosure. The vessel 120 illustrated in Fig. 1 may represent a coronary artery, for example. PVAT surrounds vessels such as the coronary arteries, and is found within, or contiguous with, the Tunica adventitia layer illustrated in Fig. 1, i.e. in the layer outside the Tunica media layer.

As described above, the document WO 2016/024128 A1 defines methods for volumetric characterization of perivascular adipose tissue using data collected by computed tomography scanning. The volumetric characterization of perivascular adipose tissue allows the inflammatory status of underlying blood vessels to be established by CT scanning. This is of use in the diagnosis, prognosis and treatment of coronary and vascular disease. The document by Antoniades C., et al., mentioned above, describes another biomarker that is derived from CT attenuation in PVAT surrounding the human coronary arteries. This document discloses a biomarker designed to capture spatial changes in PVAT's attenuation around the human coronary arteries, the Fat Attenuation Index "FAI". The FAI has predictive value in stable patients for cardiac mortality and non-fatal heart attacks.

Fig. 2 is a flowchart illustrating an example of a computer-implemented method of determining a status of vascular disease, in accordance with some aspects of the present disclosure. Fig. 3 is a schematic diagram illustrating an example of a system 200 for determining a status of vascular disease, in accordance with some aspects of the present disclosure. The system 200 includes one or more processors 210. It is noted that operations described in relation to the method that is described with reference to Fig. 2, may also be performed by the one or more processors 210 of the system 200 illustrated in Fig. 3. Likewise, operations described in relation to the one or more processors 210 of the system 200, may also be performed in the method that is described with reference to Fig. 2. With reference to Fig. 2, the computer-implemented method of determining a status of vascular disease, includes:
receiving S 110 computed tomography, CT, data 110 representing a portion of a vessel 120;
determining S 120, from the CT data 110, a spatial distribution of X-ray attenuation values across one or more concentric layers 130_{1..n} of perivascular adipose tissue, PVAT, surrounding the portion of the vessel 120;
analyzing S130 the spatial distribution of X-ray attenuation values across the one or more concentric layers 130_{1..n} to provide a spatial distribution of disease status values 140 representing a status of vascular disease around the portion of the vessel 120; and
outputting S 140 a graphical representation 150a, 150b of the spatial distribution of disease status values 140 and/or outputting S 150 a predicted time at which the status of vascular disease around the portion of the vessel 120 is expected to reach a predefined status, the predicted time being predicted based on the spatial distribution of disease status values 140.

In the above method, the provision of a spatial distribution of disease status values representing a status of vascular disease around the portion of the vessel, provides detailed information on the status of disease. This is in contrast to the provision of a single value of a biomarker for a portion of a vessel. The additional information that is provided by the spatial distribution of disease status values, may for instance be used to track changes in the disease at specific locations, thereby facilitating improved decision-making on the treatment or monitoring of the vessel. Similarly, since the outputted predicted time at which the status of vascular disease around the portion of the vessel is expected to reach a predefined status, is predicted based on the spatial distribution of disease status values, a more reliable predicted time can be provided.

With reference to the flowchart illustrated in Fig. 2, in the operation S110, CT data 110 representing a portion of a vessel 120, is received.

The CT data 110 that is received in the operation S 110 may be generated subsequent to the injection of a contrast agent into the vasculature. The contrast agent may include a substance such as iodine, or a lanthanide such as gadolinium, or another substance that provides visibility of a blood flow into which the contrast agent is injected. The CT data 110 may in general represent a static image of the vessel 120, or alternatively it may represent a temporal sequence of images of the vessel 120. In the latter case, the temporal sequence of images may be generated substantially in real-time, and the method described above may be performed substantially in real-time. Consequently, the determining operation S 120, the analyzing operation S 130, and also the outputting S 140 of the graphical representation of the spatial distribution of disease status values 140, may be performed substantially in real-time. Similarly, the operation of outputting S150 a predicted time at which the status of vascular disease around the portion of the vessel 120 is expected to reach a predefined status, may also be performed in real-time.

In general, the CT data 110 that is received in the operation S110 may be raw data, i.e. data that has not yet been reconstructed into a volumetric, or 3D, image, or it may be image data, i.e. data that has already been reconstructed into a volumetric image. The CT data may also be referred-to as volumetric data. The CT data 110 may be generated by a CT imaging system, or, as described in more below, it may be generated by rotating, or stepping the X-ray source and X-ray detector of an X-ray projection imaging system around the vessel.

A CT imaging system generates CT data by rotating, or stepping, an X-ray source-detector arrangement around an object and acquiring X-ray attenuation data for the object from multiple rotational angles with respect to the object. The CT data may then be reconstructed into a 3D image of the object. Examples of CT imaging systems that may be used to generate the CT data 110 include cone beam CT imaging systems, photon counting CT imaging systems, dark-field CT imaging systems, and phase contrast CT imaging systems. An example of a CT imaging system 220 that may be used to generate the CT data 110 that is received in the operation S110, is illustrated in Fig. 3. By way of an example, the CT data 110 may be generated by the CT 5000 Ingenuity CT scanner that is marketed by Philips Healthcare, Best, The Netherlands.

As mentioned above, the CT data 110 that is received in the operation S110 may alternatively be generated by rotating, or stepping the X-ray source and X-ray detector of an X-ray projection imaging system around the vessel 120. An X-ray projection imaging system may include a support arm such as a so-called "C-arm" that supports an X-ray source and an X-ray detector. X-ray projection imaging systems may alternatively include a support arm with a different shape to this example, such as an O-arm, for example. Other types of X-ray projection imaging systems may alternatively be used wherein the X-ray source and the X-ray detector are mounted, or supported, in a different manner. In contrast to a CT imaging system, an X-ray projection imaging system generates X-ray attenuation data for an object with the X-ray source and X-ray detector in a static position with respect to the object. The X-ray attenuation data may be referred-to as projection data, in contrast to the volumetric data that is generated by a CT imaging system. The X-ray attenuation data that is generated by an X-ray projection imaging system is typically used to generate a 2D image of the object. However, an X-ray projection imaging system may generate CT data, i.e. volumetric data, by rotating, or stepping, its X-ray source and X-ray detector around an object and acquiring projection data for the object from multiple rotational angles with respect to the object. Image reconstruction techniques may then be used to reconstruct the projection data that is obtained from the multiple rotational angles into a volumetric image in a similar manner to the reconstruction of a volumetric image using X-ray attenuation data that is acquired from a CT imaging system. Thus, the CT data 110 that is received in the operation S110, may be generated by a CT imaging system, or alternatively, it may be generated by an X-ray projection imaging system. An example of an X-ray projection imaging system that may be used to generate the CT data 110, is the Azurion 7 X-ray projection imaging system that is marketed by Philips Healthcare, Best, The Netherlands.

In some examples that are described in more detail below, the CT data 110 that is received in the operation S110 includes spectral CT data. Spectral CT data defines X-ray attenuation in an object within each of a plurality of different energy intervals DE_{1..m}. In general there may be two or more energy intervals; i.e. *m* is an integer, and *m* ≥ 2. In this regard, the spectral CT data 110 that is received in the operation S110 may be generated by a spectral CT imaging system, or by a spectral X-ray projection imaging system. In the latter case, the spectral CT data may be acquired by rotating, or stepping the X-ray source and X-ray detector of the spectral X-ray projection imaging system around the vessel, as described above. More generally, the spectral CT data 110 that is received in the operation S110 may be generated by a spectral X-ray imaging system.

The ability to generate X-ray attenuation data at multiple different energy intervals DE_{1..m} distinguishes a spectral X-ray imaging system from a conventional X-ray imaging system. By processing the data from the multiple different energy intervals, a distinction can be made between media that have similar X-ray attenuation values when measured within a single energy interval, and which would be indistinguishable in conventional X-ray attenuation data. Examples of spectral X-ray imaging systems that may be used to generate spectral CT data 110 that is received in the operation S110 include cone beam spectral X-ray imaging systems, photon counting spectral X-ray imaging systems, dark-field spectral X-ray imaging systems, and phase contrast spectral X-ray imaging systems. An example of a spectral CT imaging system that may be used to generate spectral CT data 110 that is received in the operation S110 is the Spectral CT 7500 that is marketed by Philips Healthcare, Best, The Netherlands.

In general, spectral CT data 110 may be generated by various different configurations of spectral X-ray imaging systems that include an X-ray source and an X-ray detector. The X-ray source of a spectral X-ray imaging system may include multiple monochromatic sources, or one or more polychromatic sources, and the X-ray detector of a spectral X-ray imaging system may include: a common detector for detecting multiple different X-ray energy intervals, or multiple detectors wherein each detector detects a different X-ray energy interval DE_{1..m}, or a multi-layer detector in which X-rays having energies within different X-ray energy intervals are detected by corresponding layers, or a photon counting detector that bins detected X-ray photons into one of multiple energy intervals based on their individual energies. In a photon counting detector, the relevant energy interval may be determined for each received X-ray photon by detecting the pulse height induced by electron-hole pairs that are generated in response to the X-ray photon's absorption in a direct-conversion material.

Various configurations of the aforementioned X-ray sources and detectors may be used to detect X-rays within different X-ray energy intervals DE_{1..m}. In general, discrimination between different X-ray energy intervals may be provided at the source by temporally switching the X-ray tube potential of a single X-ray source, i.e. "rapid kVp switching", or by temporally switching, or filtering, the emission of X-rays from multiple X-ray sources. In such configurations, a common X-ray detector may be used to detect X-rays across multiple different energy intervals, X-ray attenuation data for each energy interval being generated in a time-sequential manner. Alternatively, discrimination between different X-ray energy intervals may be provided at the detector by using a multi-layer detector, or a photon counting detector. Such detectors can detect X-rays from multiple X-ray energy intervals DE_{1..m} nearsimultaneously, and thus there is no need to perform temporal switching at the source. Thus, a multi-layer detector, or a photon counting detector, may be used in combination with a polychromatic source to generate X-ray attenuation data at different X-ray energy intervals DE_{1..m}.

Other combinations of the aforementioned X-ray sources and detectors may also be used to provide the spectral CT data 110. For example, in a yet further configuration, the need to sequentially switch different X-ray sources emitting X-rays at different energy intervals may be obviated by mounting X-ray source-detector pairs to a gantry at rotationally-offset positions around an axis of rotation. In this configuration, each source-detector pair operates independently, and separation between the spectral CT data for different energy intervals DE_{1..m} is facilitated by virtue of the rotational offsets of the source-detector pairs. Improved separation between the spectral CT data for different energy intervals DE_{1..m} may be achieved with this configuration by applying an energy-selective filter to the X-ray detector(s) in order to reduce the effects of X-ray scatter.

In general, the CT data 110 that is received in the operation S110 may be received via any form of data communication, including wired, optical, and wireless communication. By way of some examples, when wired or optical communication is used, the communication may take place via signals transmitted on an electrical or optical cable, and when wireless communication is used, the communication may for example be via RF or optical signals. The CT data 110 that is received in the operation S110 may be received from various sources. For example, the CT data 110 may be received from an imaging system, such as one of the imaging systems described above. Alternatively, the CT data 110 may be received from another source, such as a computer readable storage medium, the Internet, or the Cloud, for example.

The CT data 110 that is received in the operation S110 represents a portion of a vessel 120. By way of an example, the CT data 110 may represent a portion of an artery such as a coronary artery. In this example, the CT data may be used to determine a status of coronary artery disease in the artery. The CT data 110 may alternatively represent a portion of another type of vessel. For instance, the CT data 110 may alternatively represent a portion of a vein. In general, the vein, or the artery, may be disposed in any location in the body, such as in the heart, the brain, or in a peripheral region such as in the arm, the leg, and so forth.

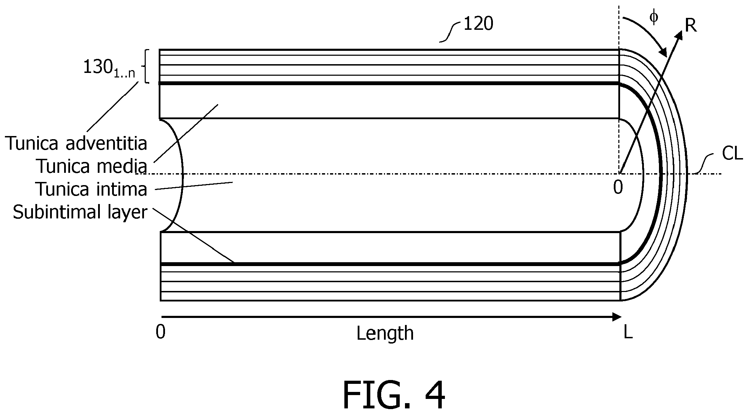

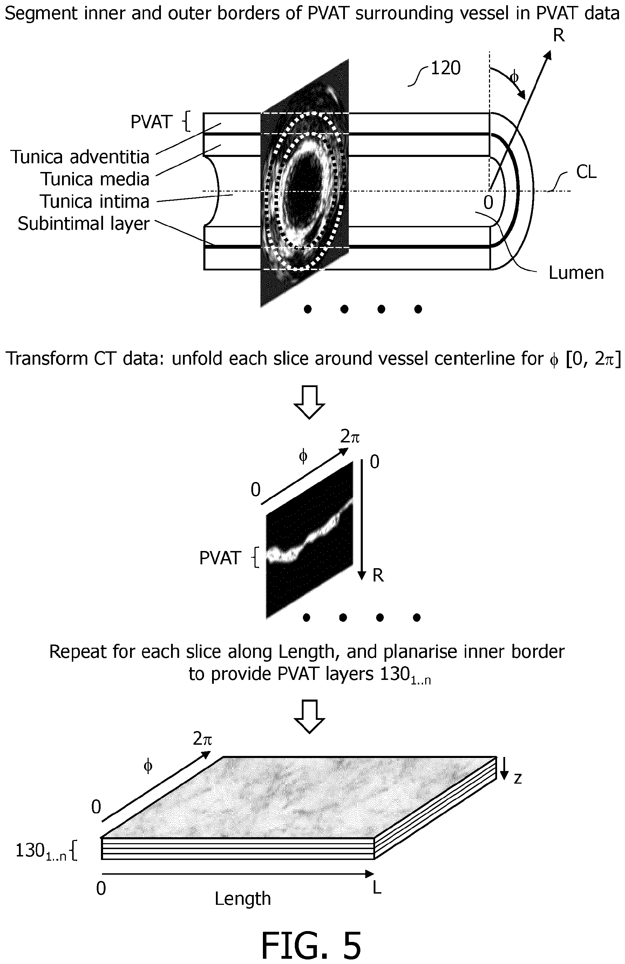

Returning to the flowchart illustrated in Fig. 2, in the operation S120, a spatial distribution of X-ray attenuation values across one or more concentric layers 130_{1..n} of perivascular adipose tissue, PVAT, surrounding the portion of the vessel 120, is determined. An example of the operation S120 is described below with reference to Fig. 4, and Fig. 5.

Fig. 4 is a schematic diagram illustrating an example of a portion of a vessel 120 including one or more concentric layers 130_{1..n} of perivascular adipose tissue, PVAT, surrounding the vessel, in accordance with some aspects of the present disclosure. The vessel 120 illustrated in Fig. 4 corresponds to the vessel 120 illustrated in Fig. 1, and in addition to the items illustrated in Fig. 1, Fig. 4 illustrates multiple concentric layers 130_{1..n} of PVAT. In this example, the PVAT is disposed in the Tunica adventitia layer. In the illustrated example, the concentric layers 130_{1..n} are defined with respect to a common center, and in this example the center is defined as the vessel centerline, CL. The concentric layers 130_{1..n} may be described as surfaces formed around the vessel centerline, CL. Each surface is bounded in an axial direction along the vessel centerline CL, by the positions 0, and L. These positions define the length of the portion of the vessel. Each surface is bounded in a rotational direction, φ, around the vessel centerline, CL, by the rotational angular range [0, 2π]. The concentric layers 130_{1..n} are bounded in a radial direction, R, by an inner radial dimension that is defined for a given rotational angle φ around the vessel centerline, CL, by the radially innermost position of the PVAT, and in an outer radial dimension that is defined for a given rotational angle φ around the vessel centerline, CL, by the radially outermost position of the PVAT. In general, there may be one or more concentric layers. In some examples there may be two or more concentric layers. If two or more concentric layers are present, the layers may have equal thicknesses. By way of some examples, there may be ten or twenty layers, or more. For instance, if there are approximately twenty layers, each layer may have a thickness the radial direction, R, of approximately 1 millimeter. By contrast, if only a few layers are provided, each layer may have a thickness of several millimeters. These values are provided purely as examples.

With continued reference to Fig. 4, in the operation S120, a spatial distribution of X-ray attenuation values across the one or more concentric layers 130_{1..n} of PVAT, is determined. This operation involves determining the X-ray attenuation along a path through each layer at each of multiple positions across each layer 130_{1..n}, i.e. the X-ray attenuation at each of multiple positions across each of the abovedefined surfaces within the limits of [0, L] along the vessel centerline, CL, and within the rotational angular range [0, 2π] in the rotational direction, φ. Examples of this operation are described with reference to Fig. 5, which is a schematic diagram illustrating an example of a technique for determining S120 a spatial distribution of X-ray attenuation values across one or more concentric layers 130_{1..n} of PVAT, in accordance with some aspects of the present disclosure.

With reference to Fig. 5, the spatial distribution of X-ray attenuation values across the one or more concentric layers 130_{1..n} of PVAT may be determined by generating virtual image slices from the CT data, the slices being arranged transversely with respect to the vessel centerline, CL. An example of such an image slice is illustrated in the upper portion of Fig. 5. In this example, the slices are arranged perpendicularly, with respect to the vessel centerline, CL. The virtual slices may be arranged at regular intervals along the vessel centerline, CL, such as at intervals of approximately 1 millimeter, for example.

The concentric layers 130_{1..n} that were described above with reference to Fig. 4, are then defined using the image slices. This operation may either be performed with the image slices as they appear in the upper portion of Fig. 5, or alternatively the CT data for each image slice may be transformed to provide an unfolded representation of the vessel, and in which case the concentric layers are defined in the unfolded representation of the vessel.

In the former case, for each image slice, the inner and outer borders of PVAT in the slice are initially defined in the slice by performing an image segmentation on the slice. As mentioned above, PVAT is identifiable in CT images via its elevated level of X-ray attenuation just outside the vessel wall. An example of the resulting segmented image is illustrated via the dotted curves in the upper portion of Fig. 5. Concentric boundaries are then defined at regular intervals in the radial direction, R, between the inner and outer borders of PVAT in each image slice in order to identify the CT data from each slice, for each of the concentric layers 130_{1..n}. For a given slice, the CT data for each layer is bounded in the radial direction by the inner and outer concentric boundaries of the slice, and bounded in the rotational direction, φ, by the rotational angular range [0, 2π]. The CT data for each layer is then obtained by concatenating the data for the layer from each of the slices in a direction along the vessel centerline, CL. This provides a spatial distribution of X-ray attenuation values across each of the layers 130_{1..n} for the portion of the vessel. From here, the method may continue with the operation S130, described below.

The latter case is illustrated in the central and lower portions of Fig. 5. In this case, an image segmentation is performed on the image slices in the same manner as described above. This defines the inner and outer borders of PVAT in each of the image slices. The CT data for each image slice is then transformed to provide an unfolded representation of the vessel 120. This operation is illustrated in the central portion of Fig. 5. This operation may be performed by applying a coordinate transformation to the CT data wherein the data from angular positions around the rotational direction, φ, is mapped to a linear axis. This maps the image slice illustrated in the upper portion of Fig. 5 to the image slice illustrated in the central portion of Fig. 5. The data from each of the image slices along the vessel centerline, CL, within the limits [0, L] is then concatenated along the direction of the vessel centerline to provide an unfolded volumetric representation of the vessel. Starting at the inner surface of the PVAT, multiple layers 130_{1..n}, each with a defined thickness, are then defined in the PVAT. This provides a spatial distribution of X-ray attenuation values across each of the layers 130_{1..n} for the portion of the vessel. From here, the method may continue with the operation S130, described below.

Owing to the irregular geometry of the vessel, and the irregular distribution of the PVAT, the concatenated data obtained from the image slices illustrated in the central portion of Fig. 5 may have a non-planar surface. This can complicate the subsequent analysis of the X-ray attenuation values across the layers. In order to avoid such complications, the inner surface of the PVAT that is defined by the inner boundaries of PVAT in the slices may be planarized. This operation may be performed by further transforming the concatenated data such that positions on the inner surface of the PVAT are mapped to a planar surface. This re-shapes the PVAT such that its inner surface is planar, as illustrated in the lower portion of Fig. 5. Multiple layers 130_{1..n}, each with a defined thickness, are then defined in the PVAT as described above, starting at the inner surface of the PVAT. This provides CT data in which the one or more concentric layers 130_{1..n} of PVAT surrounding the portion of the vessel 120 are represented as planar layers. From here, the method may continue with the operation S130, described below.

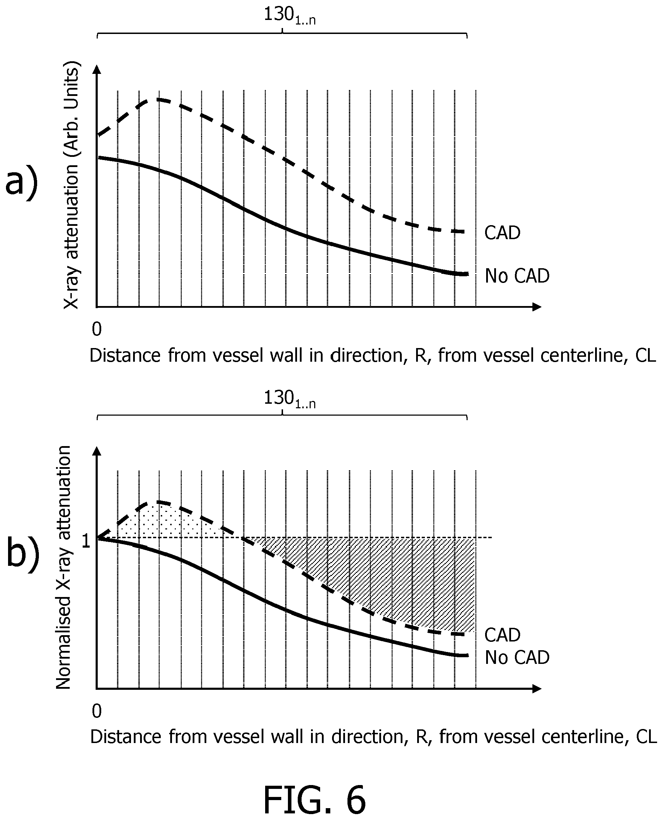

Returning to the flowchart illustrated in Fig. 2, in the operation S 130, the spatial distribution of X-ray attenuation values across the one or more concentric layers 130_{1..n} is analyzed in order to provide a spatial distribution of disease status values 140 representing a status of vascular disease around the portion of the vessel 120. In general, this operation is performed based on a gradient of the X-ray attenuation values across the one or more concentric layers 130_{1..n}. An example of the operation S 130 is described with reference to Fig. 6, which illustrates a) an example of a graph of X-ray attenuation values along a path extending in a radial direction, R, from a vessel centerline, CL, and b) an example of a normalized graph of the X-ray attenuation values illustrated in a), in accordance with some aspects of the present disclosure. The graph illustrated in Fig. 6a illustrates an example of a variation of a variation of X-ray attenuation values in the radial direction, R, illustrated in Fig. 3, for a vessel in which CAD has been detected (dashed curve), and a vessel in which no CAD has been detected (solid curve).

As described in the document by Antoniades C., et al., cited above, in the absence of vessel inflammation, the X-ray attenuation of PVAT decreases with increasing radial distance away from the vessel wall. This situation is represented by the solid curve "No CAD" illustrated in Fig. 6a. By contrast, in the presence of vessel inflammation, relatively higher X-ray attenuation values are found in PVAT. This gives rise to a relatively higher X-ray attenuation values in the vicinity of the vessel wall, followed by a relatively steeper gradient with increasing radial distance away from the vessel wall. This situation is represented by the dashed curve "CAD" illustrated in Fig. 6a.

The radial direction, R, illustrated in Fig. 6a passes approximately normally through the one or more concentric layers 130_{1..n} that were described with reference to Fig. 5. The positions of these layers 130_{1..n} are also illustrated in Fig. 6a. The value of the X-ray attenuation illustrated in Fig. 6a therefore shows the X-ray attenuation in each of the layers along a path extending in a radial direction, R, from the vessel centerline, through the concentric layers 130_{1..n}. As may be appreciated, graphs that are similar to those illustrated in Fig. 6a may be provided for different rotational directions, φ, and for different positions along the vessel centerline, CL. The shapes of these curves vary depending on the spatial distribution of CAD in the vessel.

The normalized graph of the X-ray attenuation values illustrated in Fig. 6b is obtained by normalizing the X-ray attenuation values illustrated in Fig. 6a to the X-ray attenuation value in the innermost layer of the concentric layers 130_{1..n}. Consequently, the value of the Normalized X-ray attenuation at the vessel wall in Fig. 6b corresponds to unity.

In the operation S130, the spatial distribution of disease status values 140 representing a status of vascular disease around the portion of the vessel 120, is determined. This operation may be performed by determining a spatial distribution of the values of various biomarkers. Examples of biomarkers that may be evaluated include in this operation include the Fat Attenuation Index, FAI, biomarker described in the document by Antoniades C., et al., cited above, and the Volumetric Perivascular Characterization Index, "VPCI-i", biomarker described in the documents WO 2016/024128 A1. A spatial distribution of the values of other biomarkers may alternatively be determined in this operation.

By way of an example, a spatial distribution of the Volumetric Perivascular Characterization Index, "VPCI-i", biomarker described in the documents WO 2016/024128 A1, is now described with reference to Fig. 6. In this example, the determining S120 operation, and the analyzing S130 operation, that were described above with reference to Fig. 2, are performed for a plurality of concentric layers 130_{1..n} of PVAT. In this example, the analyzing operation S 130 includes:
normalizing the spatial distribution of X-ray attenuation values across the one or more concentric layers 130_{1..n} of PVAT surrounding the portion of the vessel 120, based on the X-ray attenuation values across an innermost layer of the one or more concentric layers 130_{1..n};
integrating the normalized X-ray attenuation values along paths extending radially outwards through the plurality of concentric layers 130_{1..n} of PVAT from n centerline of the vessel 120; and
comparing the integrated normalized X-ray attenuation values with a threshold value to provide the spatial distribution of disease status values 140 representing a status of vascular disease around the portion of the vessel 120; and
wherein the integrating comprises assigning negative values to normalized X-ray attenuation values exceeding the normalized X-ray attenuation value in the innermost layer along the corresponding path, and assigning positive values to normalized X-ray attenuation values below the normalized X-ray attenuation value in the innermost layer along the corresponding path.

The above operations may be performed using the CT data representing the vessel as it appears in the upper portion of Fig. 5, i.e. the vessel in its actual shape, or alternatively, they may be performed using the CT data representing the vessel in an unfolded, and optionally further planarized, state, i.e. using the CT data representing the vessel as it appears in the central, or lower portions of Fig. 5.

In the above operations, the provision of the spatial distribution of disease status values 140 involves integrating normalized X-ray attenuation values along paths extending radially outwards through the plurality of concentric layers 130_{1..n} of PVAT from a centerline of the vessel 120. With reference to the upper portion of Fig. 5, the paths may be defined at specified intervals in the rotational direction, φ, and at specified positions along the vessel centerline, CL. For instance, the paths may be defined at regular intervals in the rotational direction, φ, and at regular intervals along the vessel centerline, CL. If the CT data representing the vessel as it appears in the upper portion of Fig. 5, is used, the normalized X-ray attenuation values may be obtained by normalizing the X-ray attenuation values along each path to the X-ray attenuation value in the innermost layer, as was described above with reference to Fig. 6a and Fig. 6b. The integration operation then amounts to performing an integral of the values along each path using the normalized X-ray attenuation values. If the CT data representing the vessel as it appears in the lower portion of Fig. 5, is used, the paths in this unfolded representation of the vessel extend normally through the layers 130_{1..n}. In this case, the normalized X-ray attenuation values are again obtained by normalizing the X-ray attenuation values along the path to the X-ray attenuation value in the innermost layer, as described above with reference to Fig. 6b. This amounts to normalizing the X-ray attenuation values along the thickness direction, z, of the concentric layers 130_{1..n} to the value of the X-ray attenuation at the corresponding position on the innermost layer. The integration operation then amounts to performing an integral of the values along the path, i.e. along the thickness direction, z, of the layers, using the normalized X-ray attenuation values.

The integration operation also includes assigning negative values to normalized X-ray attenuation values exceeding the normalized X-ray attenuation value in the innermost layer along the corresponding path, and assigning positive values to normalized X-ray attenuation values below the normalized X-ray attenuation value in the innermost layer along the corresponding path. With reference to Fig. 6b), this results in X-ray attenuation values that are below unity being added to the integrated value, and X-ray attenuation values that exceed unity being subtracted from the integrated value. Thus, the integrated value for each path represents the difference between the area shaded with hatching in Fig. 6b that is above the line "Normalized X-ray attenuation = 1", and the area shaded with dots.

The result of the integration described above is to provide a spatial distribution of integrated normalized X-ray attenuation values wherein each integrated value represents the integral along a different path. In the comparing operation, the integrated value for each path is then compared with a threshold value to provide a disease status value for the path. The disease status value for the path may be an analogue value, or a digital value, such as CAD, or No CAD, for example. The threshold value may be determined from clinical studies that e.g. separate vessels that include CAD, from vessels that do not include CAD. Thus, by performing the comparing operation for each of the paths, a spatial distribution of disease status values 140 representing a status of vascular disease around the portion of the vessel 120, is provided.

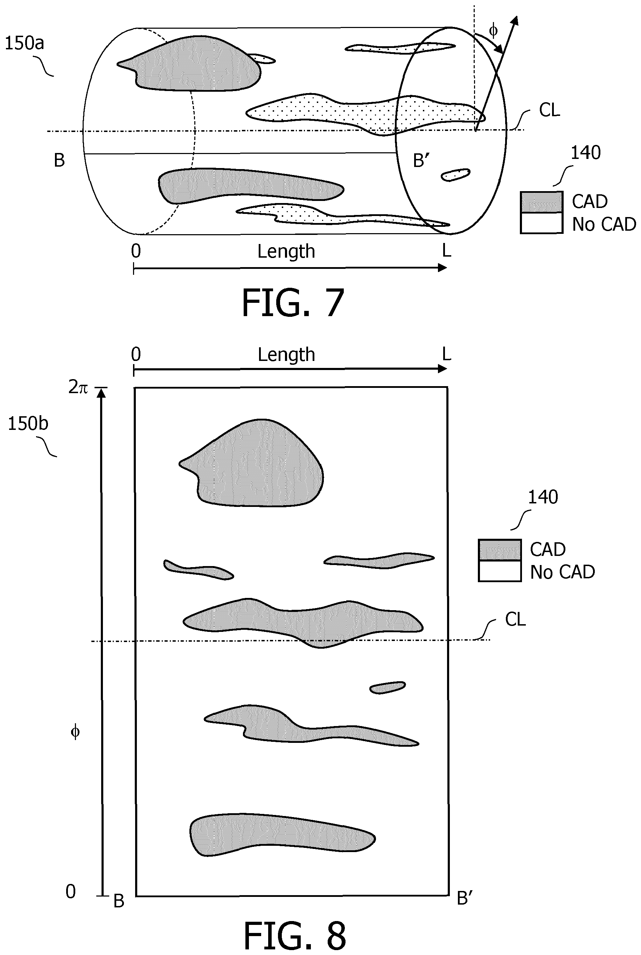

Returning to the flowchart illustrated in Fig. 2, in the operation S 120, a graphical representation 150a, 150b of the spatial distribution of disease status values 140 is then outputted in the operation S 140. The graphical representation 150a, 150b may be outputted to a display, such as the display 230 illustrated in Fig. 3, for example. Examples of such graphical representations 150a, 150b are described below with reference to Fig. 7 - Fig. 10. Alternatively, or additionally, in the operation S 150 a predicted time at which the status of vascular disease around the portion of the vessel 120 is expected to reach a predefined status, is outputted. The predicted time is predicted based on the spatial distribution of disease status values 140.

In one example, a graphical representation of the spatial distribution of disease status values 140 representing the status of vascular disease around the portion of the vessel 120, is outputted. In this example, the graphical representation 150a comprises a projection of the spatial distribution of disease status values 140 onto a surface of revolution formed around the portion of the vessel 120. This example is described with reference to Fig. 7, which is a schematic diagram illustrating a first example of a graphical representation 150a of a spatial distribution of disease status values 140, in accordance with some aspects of the present disclosure. In the example illustrated in Fig. 7, the surface of revolution comprises a cylindrical surface. The disease status values 140 are projected along the paths along which the integration was performed and the corresponding disease status values 140 is displayed on the cylindrical surface of the at the point of intersection between the path and the cylindrical surface. In the example illustrated in Fig. 7, dark-shaded areas represent regions of CAD, and light-shaded regions represent regions where CAD is not present. The disease status values 140 in this example may alternatively be displayed in a different manner, such as via different colors. In addition to the graphical representation illustrated in Fig. 7, an image illustrating the portion of the vessel 120 may also be displayed. For instance, a reconstructed image of the portion of the vessel may be generated from the CT data 110 and displayed within the cylindrical surface. This helps to convey the correspondence between the vessel anatomy and the disease status values. A user may be provided with the ability to manipulate the graphical representation in order to obtain a different perspective of the disease status values 140.

In another example, a graphical representation of the spatial distribution of disease status values 140 representing the status of vascular disease around the portion of the vessel 120, is outputted. In this example, the graphical representation 150b comprises a projection the spatial distribution of disease status values 140 onto an unfolded surface, the unfolded surface being an unfolded representation of a surface of revolution formed around the portion of the vessel 120. This example is illustrated in Fig. 8. which is a schematic diagram illustrating a second example of a graphical representation 150b of a spatial distribution of disease status values 140, in accordance with some aspects of the present disclosure. As compared to the example illustrated in Fig. 7, the cylindrical surface illustrated in Fig. 7 has been unfolded. This graphical representation may be generated by virtually cutting the cylindrical surface along the line B - B', and unfolding the surface around the centerline, CL, to map the cylindrical surface of Fig. 7 onto a planar surface as illustrated in Fig. 8. As compared to the graphical representation 150a illustrated in Fig. 7, the graphical representation 150b provides a less-obstructed view of the disease status values 140. The graphical representation 150b illustrated in Fig. 8 may alternatively be generated directly from the data illustrated in the lower portion of Fig. 5 by displaying the disease status values 140 directly on the PVAT.

As mentioned above, in the operation S 150 a predicted time at which the status of vascular disease around the portion of the vessel 120 is expected to reach a predefined status, is outputted. The predicted time is predicted based on the spatial distribution of disease status values 140. The operation S 150 may be performed alternatively, or additionally, to the operation S 140.

In this example, the predefined status is defined by one or more of:
a value from the spatial distribution of disease status values 140 exceeding a predetermined threshold value;
an onset of a symptom of vascular disease;
a physiological parameter of the vessel 120 meeting a predetermined threshold condition; and
a value of a vascular disease risk metric exceeding a predetermined threshold value.

Since in this example, the predicted time is predicted based on the spatial distribution of disease status values 140, a more accurate prediction may be obtained. Examples of symptoms, the onset of which may be predicted in accordance with this example, include pain, a skin infection, skin ulcers, skin discoloration, numbness, stroke, nausea, blood clots, chest pain, irregular heartbeat, shortness of breath, and so forth. Examples of physiological parameters that may be used to define the predefined status include a measurement of vessel geometry such as vessel area, or vessel diameter, a measurement of a blood flow parameter such as blood flow rate, fractional flow reserve "FFR", instantaneous wavefree ratio "iFR", Coronary Flow Reserve "CFR", and so forth. Examples of vascular disease risk metrics include a risk of CAD, a risk of peripheral artery disease "PAD", the WIFI score, the GLASS score, the Villata score, the Framingham Risk Score, JBS3, and so forth.

In one example, the predicted time may be determined by inputting the spatial distribution of disease status values 140 into a neural network that is trained to predict a time at which a status of vascular disease, such as described in the examples described above, is expected to reach a predefined status, The neural network may be trained using training data comprising spatial distributions of disease status values 140, each spatial distribution having a corresponding ground truth value for the time at which the status of the relevant vascular disease, is reached. The training data may include some thousands, or more spatial distributions and corresponding ground truth values. The training data may be curated from historic clinical investigations performed on different subjects.

Variations of the method described above with reference to Fig. 2, are also contemplated.

In one example, the CT data 110 comprises spectral CT data. Spectral CT data defines X-ray attenuation in media within multiple different energy intervals. As mentioned above, by processing the data from the multiple different energy intervals, a distinction can be made between media that have similar X-ray attenuation values when measured within a single energy interval, and which would be indistinguishable in conventional X-ray attenuation data.

In this example, the CT data 110 comprises spectral CT data defining X-ray attenuation in the portion of the vessel 120 within each of a plurality of different energy intervals. The operation of determining S 120 a spatial distribution of X-ray attenuation values across one or more concentric layers 130_{1..n} of PVAT surrounding the portion of the vessel 120, comprises extracting from the spectral CT data, PVAT attenuation data representing a spatial distribution of the X-ray attenuation values in PVAT across the one or more concentric layers 130_{1..n} of PVAT surrounding the portion of the vessel 120. The operation of analyzing S 130 the spatial distribution of X-ray attenuation values, is performed using the extracted PVAT attenuation data.

In this example, the spectral CT data may be generated by various different configurations of spectral X-ray imaging systems, as described above. The use of spectral CT data facilitates improved distinction between the X-ray attenuation arising from PVAT and the X-ray attenuation arising from other media that may be present in the vicinity of the vessel 120. This, in turn, provides a more reliable determination of the spatial distribution of disease status values 140 in the operation S130.

In another example the CT data that is received in the operation S110 represents the vessel at multiple different points in time. The CT data from the different points in time is then mutually registered. This enables changes in the spatial distributions of disease status values 140 over time to be identified. This example therefore facilitates an improved understanding of the progression of disease in the vessel.

In this example, the CT data 110 represents the portion of the vessel 120 at each of a plurality of points in time to, t₁; the determining S120 operation, and the analyzing S130 operation, are performed for the CT data representing the portion of the vessel 120 at each point in time t₀, t₁; and the method described with reference to Fig. 2 includes:
mutually registering the spatial distributions of X-ray attenuation values representing the portion of the vessel 120 at each point in time t₀, t₁; and
wherein the outputting S140 a graphical representation of the spatial distribution of disease status values 140, comprises outputting the mutually registered spatial distributions of disease status values 140 representing the status of vascular disease around the portion of the vessel 120 at each point in time t₀, t₁ and/or outputting a spatial distribution of disease status values representing a change in the status of vascular disease around the portion of the vessel 120 between two of the points in time t₀, t₁, the change in the status of vascular disease being calculated using the mutually registered spatial distributions at the two points in time.

In this example, the points in time may be separated by some days, weeks, or months, for example. The points in time may represent the times of initial clinical investigations, or follow-up investigations on the vessel, for example.

The mutually registered spatial distributions of disease status values 140 that are outputted in this example may be outputted by overlaying the spatial distributions. Various known techniques may be used to overlay the spatial distributions.

The spatial distribution of disease status values representing a change in the status of vascular disease that is outputted in accordance with this example, may be generated by performing a subtraction operation on the mutually registered spatial distributions. The spatial distribution of disease status values representing a change in the status of vascular disease, may indicate the difference between the areas of regions that have been identified as representing CAD, for example.

The registration that is performed in accordance with this example may be performed using various techniques. In general, the registration may be a rigid, or an affine, or a deformable registration. Various image registration techniques may be used, including intensity-based image registration techniques, feature-based image registration techniques, neural networks, and so forth. The image registration may be performed with the vessel represented in its actual shape, i.e. as it appears in a reconstruction of the received CT data 110, or alternatively it may be performed using an unfolded representation of the vessel, such as the unfolded representations described with reference to Fig. 5.

In one example, the registration operation includes identifying a centerline of the vessel in the CT data at each point in time. The vessel centerlines are then registered in order to mutually register the spatial distributions of X-ray attenuation values at each point in time. In this example, the operation of determining S120 a spatial distribution of X-ray attenuation values across one or more concentric layers 130_{1..n} of PVAT, includes identifying a centerline of the portion of the vessel 120 in the CT data 110 at each of the plurality of points in time, and the operation of mutually registering the spatial distributions of X-ray attenuation values, comprises mutually registering the centerlines of the portions of the vessel 120 at each of the plurality of points in time.

The vessel centerline provides a reliable reference position in the vessel with which to perform the registration. The centerline of the vessel may be identified by identifying a lumen of the vessel in the CT data, defining a centerline of the lumen in the vessel, and defining the centerline of the lumen as the vessel centerline, CL. The centerline of the lumen of the vessel may be defined as a line passing through the geometric centers of a plurality of planes transversely intersecting the vessel lumen. The vessel centerline may be defined in this manner using the image slices illustrated in the upper portion of Fig. 5.

In a related example, the spatial distribution of X-ray attenuation values along the centerline of the vessel 120 is used to normalize the spatial distribution of X-ray attenuation values in the CT data. In this example, the operation of determining S120 a spatial distribution of X-ray attenuation values across one or more concentric layers 130_{1..n} of PVAT, includes:
determining a spatial distribution of X-ray attenuation values along the centerline of the portion of the vessel 120 at a selected one of the points in time; and
normalizing the spatial distribution of X-ray attenuation values across the one or more concentric layers 130_{1..n} of PVAT surrounding the portion of the vessel 120, for the CT data 110 representing the vessel 120 at one or more of the other points in time, based on the determined spatial distribution.

The X-ray attenuation values in the CT data for a region of interest can vary depending on factors such as the CT imaging system used to acquire the CT data, as well as the settings of values such as the X-ray energy, CT dose, and gantry rotation speed, and so forth. Moreover, the spatial distribution of X-ray attenuation values can also vary along the vessel due to natural flow characteristics in the vessel, a natural tapering of the vessel, and so forth. Thus, by using the spatial distribution of X-ray attenuation values along the centerline of the vessel to normalize the data, this example provides a reliable spatial correction for such effects. Consequently, it reduces the impact of variations arising from differences in the manner in which the CT data is acquired. In this example, the selected point in time may be a time of a first image in the temporal series, or a time of another image. The spatial distribution of X-ray attenuation values along the vessel centerline from the selected image is then applied to the one or more further images so as to provide the same intensity distribution along the vessel centerline.

In one example, the registration operation is performed using anatomical landmarks. In this example, the operation of mutually registering the spatial distributions of X-ray attenuation values, comprises:
identifying one or more anatomical landmarks in each spatial distribution of X-ray attenuation values; and
mutually registering the spatial distributions of X-ray attenuation values based on the identified one or more anatomical landmarks.

In this example, the anatomical landmarks may be identified by performing an image segmentation operation on the CT data. Various image segmentation algorithms may be used for this purpose, including model-based segmentation, watershed-based segmentation, region growing, level sets, graphcuts, and so forth. A neural network may also be trained to segment the CT data 110 in order to identify anatomical landmarks. The one or more anatomical landmarks that may be identified in this example may include at least one of: a branch in the vessel 120, a lesion in the vessel 120, for example.

In one example, the portion of the vessel may be automatically identified in the CT data. This provides a reliable identification of the portion of the vessel, and ensures that the same portion of the vessel is analyzed in the mutually registered spatial distributions of X-ray attenuation values. This, in-turn facilitates an accurate evaluation of changes in the spatial distribution of disease status values 140 over time. In this example, the operation of determining S120 a spatial distribution of X-ray attenuation values across one or more concentric layers 130_{1..n} of PVAT, includes identifying the portion of the vessel 120 in the CT data 110 at each of the plurality of points in time. The portions of the vessel may be identified based on the positions of landmarks in the images, such as a branch in the vessel, or a lesion in the vessel, for example. For instance, the portion of the vessel may be identified as the portion of the vessel between two branches.

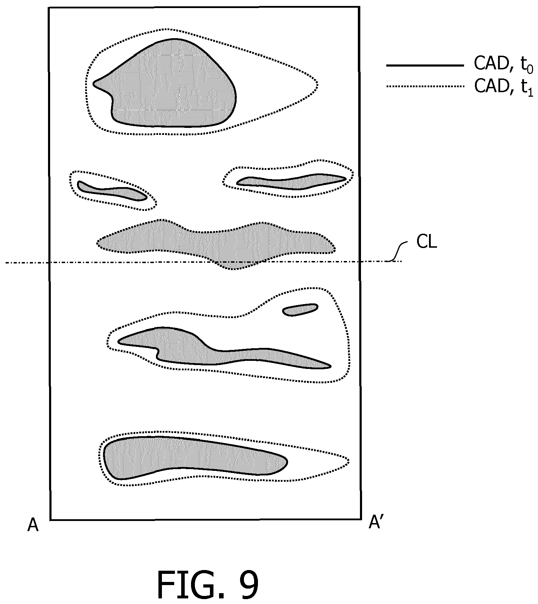

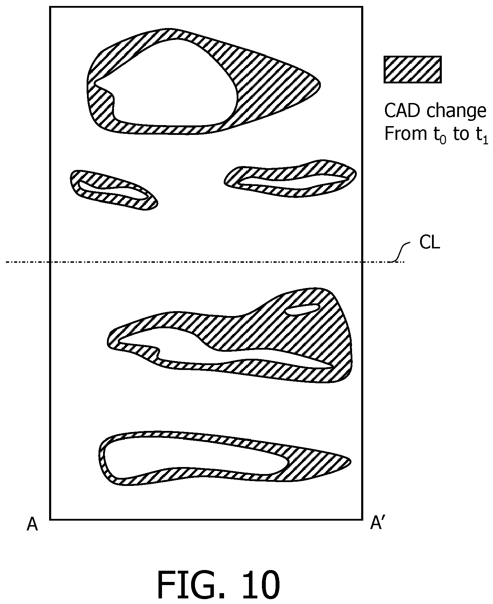

An example in which the image registration is performed using an infolded representation of the vessel is described with reference to Fig. 9, and Fig. 10. In this example, the method described with reference to Fig. 2 includes:
transforming the received CT data 110 to provide an unfolded representation of the vessel 120 wherein the one or more concentric layers 130_{1..n} of PVAT surrounding the portion of the vessel 120 are represented as planar layers; and
wherein the determining S120 a spatial distribution of X-ray attenuation values across one or more concentric layers 130_{1..n} of PVAT surrounding the portion of the vessel 120, is performed using the transformed CT data; and
wherein the mutually registering the spatial distributions of X-ray attenuation values representing the portion of the vessel 120 at each point in time t₀, t₁, is performed using the spatial distributions of X-ray attenuation values that are determined from the transformed CT data.

In this example, the registration is performed with the unfolded representation of the vessel 120 described with reference to the lower portion of Fig. 5. Registering the spatial distributions in this form reduces irregularities that may appear in the spatial distribution as a consequence of changes in vessel shape between the points in time. Such irregularities might otherwise confound the evaluation of changes in the spatial distribution of disease status values 140 over time.

Fig. 9 is a schematic diagram illustrating an example of a graphical representation of mutually registered spatial distributions of disease status values 140 representing the status of vascular disease around the portion of the vessel 120 at two different points in time t₀, t₁. In Fig. 9, the shaded regions bounded by the solid curve illustrate the spatial distribution of disease status values 140, in this example the extent of CAD, at time to. The regions bounded by the dotted curve illustrate the extent of CAD, at time t₁. The graphical representation illustrated in Fig. 9 provides a clear illustration of changes in the spatial distribution of disease status values 140, without the confounding effects of vessel shape.

Fig. 10 is a schematic diagram illustrating an example of a graphical representation of a spatial distribution of disease status values representing a change in the status of vascular disease around the portion of the vessel 120 between two points in time t₀, t₁, in accordance with some aspects of the present disclosure. The hatched regions in Fig. 10 illustrate the change in spatial distribution of CAD in the time period t₀ to t₁. These regions are determined by subtracting the areas of the regions representing CAD at the times t₀ and t₁ illustrated in Fig. 9.

In another example, one or more recommended treatment or monitoring steps are determined for the vessel 120 based on the spatial distribution of disease status values 140. The one or more recommended treatment or monitoring steps may be referred-to as a treatment plan. In this example, the method described with reference to Fig. 2 includes:
determining one or more recommended treatment or monitoring steps for the vessel 120, based on the spatial distribution of disease status values 140;
outputting the one or more recommended treatment or monitoring steps.

Examples of treatment steps in accordance with this example include a recommendation for a treatment of the vessel with a drug, recommendation for a treatment of the vessel with a stent, a recommendation for more exercise, and so forth. Examples of monitoring steps in accordance with this example include a recommendation for blood pressure monitoring, a recommendation to visit to cardiologist, and so forth.

In one example, the recommended treatment or monitoring step(s) are determined from a database. In this example, the database stores multiple reference graphical representations and corresponding recommended treatment or monitoring step(s) from historic investigations. For a given graphical representation, the database is searched in order to identify a matching graphical representation, and consequently to provide the recommended treatment or monitoring step(s). The matching graphical representation may be identified by comparing the graphical representation with the reference graphical representations based on the value of a similarity metric such as the dice coefficient, cross entropy, and so forth.

In another example, the recommended treatment or monitoring step(s) are determined by inputting the spatial distribution of disease status values 140 into a predictive model. The predictive model may be based on artificial intelligence, machine learning, or a decision tree, for example. The predictive model may be trained using training data, and wherein the training data represents, for each of a plurality of vessels, a spatial distribution of disease status values 140 representing a status of vascular disease around the portion of the vessel 120, and corresponding ground truth data representing one or more historic recommended treatment or monitoring steps for the vessel 120.

The operation of determining one or more recommended treatment or monitoring steps for the vessel 120 may also be based on additional information.

In one example, patient data relating to the vessel 120 is received, and the operation of determining one or more recommended treatment or monitoring steps for the vessel 120, is based further on the patient data. The patient data may represent factors such as patient age, body mass index, gender, blood pressure, blood cholesterol value, an indication of family history of vascular disease, an indication of smoking, diabetes, or dyspnea, and so forth.

In another example, additional data is extracted from the CT data and used to determine one or more recommended treatment or monitoring steps for the vessel 120. In this example, a distribution of plaque in the vessel 120 and/or a value of one or more blood flow parameters for the vessel 120, is determined from the received CT data 110, and the operation of determining one or more recommended treatment or monitoring steps for the vessel 120, is based further on the distribution of plaque, or the determined value of the one or more blood flow parameters, respectively.

In this example, the additional data may be provided as a further input to the predictive model, and corresponding additional training data may be used to train the predictive model to predict the one or more recommended treatment or monitoring steps for the vessel 120. The distribution of plaque in the vessel may be identified based on the values of the X-ray attenuation in the CT data 110. Improved isolation of plaque regions in the CT data may be achieved by providing the CT data 110 as spectral CT data 110.

In one example, an X-ray projection imaging system is used to navigate an intravascular treatment device to the portion of the vessel, and the graphical representation is used to position the intravascular treatment device in the vessel. In this example, the method described with reference to Fig. 2 includes:
receiving X-ray image data representing an intravascular treatment device in the vessel 120;
registering the X-ray image data to the graphical representation of the spatial distribution of disease status values 140; and
indicating a position of the intravascular treatment device in the graphical representation.

In this example, the X-ray image data may be provided by an X-ray projection imaging system. The registration may be performed by registering the X-ray image data to the CT image data. The position of the intravascular treatment device in the graphical representation may be indicated in various ways, such as by displaying an overlay of the X-ray image data and the graphical representation 150a, 150b, or by displaying a marker, for example. By indicating the position of the intravascular treatment device in the graphical representation, this example facilitates accurate positioning of the treatment device with respect to the diseased regions in the vessel. Thus, a treatment may be selectively delivered to a diseased region. The location of the treatment device in the graphical representation at the time of a delivered treatment may also be recorded so as to provide a record of the treatment. Examples of treatments that may be delivered to the vessel in accordance with this example include the injection of a drug such as Dexamethasone, and Canakinumab. An example of an intravascular treatment device that may be used in accordance with this example is the Bullfrog Micro-Infusion Device marketed by Mercator MedSystems, Inc, CA, USA.

In another example, a computer program product is provided. The computer program product comprises instructions which when executed by one or more processors, cause the one or more processors to carry out a method of determining a status of vascular disease. The method comprises:
receiving S 110 computed tomography, CT, data 110 representing a portion of a vessel 120;
determining S 120, from the CT data 110, a spatial distribution of X-ray attenuation values across one or more concentric layers 130_{1..n} of perivascular adipose tissue, PVAT, surrounding the portion of the vessel 120;
analyzing S130 the spatial distribution of X-ray attenuation values across the one or more concentric layers 130_{1..n} to provide a spatial distribution of disease status values 140 representing a status of vascular disease around the portion of the vessel 120; and
outputting S 140 a graphical representation 150a, 150b of the spatial distribution of disease status values 140 and/or outputting S 150 a predicted time at which the status of vascular disease around the portion of the vessel 120 is expected to reach a predefined status, the predicted time being predicted based on the spatial distribution of disease status values 140.

In another example, a system 200 for determining a status of vascular disease, is provided. The system includes one or more processors 210 configured to:
receive S 110 computed tomography, CT, data 110 representing a portion of a vessel 120;
determine S 120, from the CT data 110, a spatial distribution of X-ray attenuation values across one or more concentric layers 130_{1..n} of perivascular adipose tissue, PVAT, surrounding the portion of the vessel 120;
analyze S130 the spatial distribution of X-ray attenuation values across the one or more concentric layers 130_{1..n} to provide a spatial distribution of disease status values 140 representing a status of vascular disease around the portion of the vessel 120; and
output S140 a graphical representation 150a, 150b of the spatial distribution of disease status values 140 and/or output S150 a predicted time at which the status of vascular disease around the portion of the vessel 120 is expected to reach a predefined status, the predicted time being predicted based on the spatial distribution of disease status values 140.

An example of the system 200 is illustrated in Fig. 3. It is noted that the system 200 may also include one or more of: a CT imaging system 220 for generating the CT data 110 that is received in the operation S110; a monitor 230 for displaying the outputted graphical representation 150a, 150b, reconstructed CT images generated from the CT data 110, and so forth; a patient bed 240; an injector (not illustrated in Fig. 2) for injecting a contrast agent into the vasculature; and a user input device configured to receive user input, such as a keyboard, a mouse, a touchscreen, and so forth.

The above examples are to be understood as illustrative of the present disclosure, and not restrictive. Further examples are also contemplated. For instance, the examples described in relation to computer-implemented methods, may also be provided by the corresponding computer program product, or by the corresponding computer-readable storage medium, or by the corresponding system 200. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. A computer-implemented method of determining a status of vascular disease, the method comprising:
receiving (S110) computed tomography, CT, data (110) representing a portion of a vessel (120);
determining (S120), from the CT data (110), a spatial distribution of X-ray attenuation values across one or more concentric layers (130_{1..n}) of perivascular adipose tissue, PVAT, surrounding the portion of the vessel (120);
analyzing (S130) the spatial distribution of X-ray attenuation values across the one or more concentric layers (130_{1..n}) to provide a spatial distribution of disease status values (140) representing a status of vascular disease around the portion of the vessel (120); and
outputting (S140) a graphical representation (150a, 150b) of the spatial distribution of disease status values (140) and/or outputting (S150) a predicted time at which the status of vascular disease around the portion of the vessel (120) is expected to reach a predefined status, the predicted time being predicted based on the spatial distribution of disease status values (140).

2. The computer-implemented method according to claim 1, wherein the CT data (110) comprises spectral CT data defining X-ray attenuation in the portion of the vessel (120) within each of a plurality of different energy intervals; and
wherein the determining (S120) a spatial distribution of X-ray attenuation values across one or more concentric layers (130_{1..n}) of PVAT surrounding the portion of the vessel (120), comprises extracting from the spectral CT data, PVAT attenuation data representing a spatial distribution of the X-ray attenuation values in PVAT across the one or more concentric layers (130_{1..n}) of PVAT surrounding the portion of the vessel (120); and
wherein the analyzing (S130) the spatial distribution of X-ray attenuation values, is performed using the extracted PVAT attenuation data.

3. The computer-implemented method according to claim 1 or claim 2, wherein the CT data (110) represents the portion of the vessel (120) at each of a plurality of points in time (t₀, t₁); and
wherein the determining (S120), and the analyzing (S130), are performed for the CT data representing the portion of the vessel (120) at each point in time (t₀, t₁); and
wherein the method further comprises:
mutually registering the spatial distributions of X-ray attenuation values representing the portion of the vessel (120) at each point in time (t₀, t₁); and
wherein the outputting (S140) a graphical representation of the spatial distribution of disease status values (140), comprises outputting the mutually registered spatial distributions of disease status values (140) representing the status of vascular disease around the portion of the vessel (120) at each point in time (t₀, t₁) and/or outputting a spatial distribution of disease status values representing a change in the status of vascular disease around the portion of the vessel (120) between two of the points in time (t₀, t₁), the change in the status of vascular disease being calculated using the mutually registered spatial distributions of X-ray attenuation values at the two points in time.

4. The computer-implemented method according to claim 3, wherein the method further comprises transforming the received CT data (110) to provide an unfolded representation of the vessel (120) wherein the one or more concentric layers (130_{1..n}) of PVAT surrounding the portion of the vessel (120) are represented as planar layers; and
wherein the determining (S120) a spatial distribution of X-ray attenuation values across one or more concentric layers (130_{1..n}) of PVAT surrounding the portion of the vessel (120), is performed using the transformed CT data; and
wherein the mutually registering the spatial distributions of X-ray attenuation values representing the portion of the vessel (120) at each point in time (t₀, t₁), is performed using the spatial distributions of X-ray attenuation values that are determined from the transformed CT data.

5. The computer-implemented method according to claim 3 or claim 4, wherein the mutually registering the spatial distributions of X-ray attenuation values, comprises:
identifying one or more anatomical landmarks in each spatial distribution of X-ray attenuation values; and
mutually registering the spatial distributions of X-ray attenuation values based on the identified one or more anatomical landmarks.

6. The computer-implemented method according to claim 5, wherein the one or more anatomical landmarks represents at least one of: a branch in the vessel (120), a lesion in the vessel (120).

7. The computer-implemented method according to any one of claims 3-6, wherein the determining (S120) comprises identifying the portion of the vessel (120) in the CT data (110) at each of the plurality of points in time.

8. The computer-implemented method according to any one of claims 3-7, wherein the determining (S120) comprises identifying a centerline of the portion of the vessel (120) in the CT data (110) at each of the plurality of points in time; and
wherein the mutually registering the spatial distributions of X-ray attenuation values, comprises mutually registering the centerlines of the portions of the vessel (120) at each of the plurality of points in time;
and/or
wherein the determining (S120) further comprises:
determining a spatial distribution of X-ray attenuation values along the centerline of the portion of the vessel (120) at a selected one of the points in time; and
normalizing the spatial distribution of X-ray attenuation values across the one or more concentric layers (130_{1..n}) of PVAT surrounding the portion of the vessel (120), for the CT data (110) representing the vessel (120) at one or more of the other points in time, based on the determined spatial distribution.

9. The computer-implemented method according to any one of claims 1-8, wherein the graphical representation of the spatial distribution of disease status values (140) representing the status of vascular disease around the portion of the vessel (120) comprises a projection of the spatial distribution of disease status values (140) onto a surface of revolution formed around the portion of the vessel (120); or
wherein the graphical representation of the spatial distribution of disease status values (140) representing the status of vascular disease around the portion of the vessel (120) comprises a projection the spatial distribution of disease status values (140) onto an unfolded surface, the unfolded surface being an unfolded representation of a surface of revolution formed around the portion of the vessel (120).

10. The computer-implemented method according to any one of claims 1-9, wherein the method further comprises:
receiving X-ray image data representing an intravascular treatment device in the vessel (120);
registering the X-ray image data to the graphical representation of the spatial distribution of disease status values (140); and
indicating a position of the intravascular treatment device in the graphical representation.

11. The computer-implemented method according to claim 1 or claim 2, wherein the method comprises outputting (S150) a predicted time at which the status of vascular disease around the portion of the vessel (120) is expected to reach a predefined status, and wherein the predefined status is defined by one or more of:
a value from the spatial distribution of disease status values (140) exceeding a predetermined threshold value;
an onset of a symptom of vascular disease;
a physiological parameter of the vessel (120) meeting a predetermined threshold condition; and
a value of a vascular disease risk metric exceeding a predetermined threshold value.

12. The computer-implemented method according to any one of claims 1 - 11, wherein the method further comprises:
determining one or more recommended treatment or monitoring steps for the vessel (120), based on the spatial distribution of disease status values (140);
outputting the one or more recommended treatment or monitoring steps.

13. The computer-implemented method according to claim 12, further comprising:
determining, from the received CT data (110), a distribution of plaque in the vessel (120) and/or a value of one or more blood flow parameters for the vessel (120); and
wherein the determining one or more recommended treatment or monitoring steps for the vessel (120), is based further on the distribution of plaque, or the determined value of the one or more blood flow parameters, respectively.

14. The computer-implemented method according to any claim 12 or claim 13, wherein the determining one or more recommended treatment or monitoring steps for the vessel (120), comprises inputting the spatial distribution of disease status values (140) into a predictive model.

15. The computer-implemented method according to any one of claims 1 - 14, wherein the determining (S120), and the analyzing (S130), are performed for a plurality of concentric layers (130_{1..n}) of PVAT, and wherein the analyzing (S130) further comprises:
normalizing the spatial distribution of X-ray attenuation values across the one or more concentric layers (130_{1..n}) of PVAT surrounding the portion of the vessel (120), based on the X-ray attenuation values across an innermost layer of the one or more concentric layers (130_{1..n});
integrating the normalized X-ray attenuation values along paths extending radially outwards through the plurality of concentric layers (130_{1..n}) of PVAT from a centerline of the vessel (120); and
comparing the integrated normalized X-ray attenuation values with a threshold value to provide the spatial distribution of disease status values (140) representing a status of vascular disease around the portion of the vessel (120); and
wherein the integrating comprises assigning negative values to normalized X-ray attenuation values exceeding the normalized X-ray attenuation value in the innermost layer along the corresponding path, and assigning positive values to normalized X-ray attenuation values below the normalized X-ray attenuation value in the innermost layer along the corresponding path.
